**Europäisches Patentamt**

**European Patent Office**

**Office européen des brevets**

(19)

(11) Veröffentlichungsnummer : **0 163 165**
**B1**

(12) **EUROPÄISCHE PATENTSCHRIFT**

(45) Veröffentlichungstag der Patentschrift :
17.08.88

(51) Int. Cl.⁴ : **A 61 M 25/00**

(21) Anmeldenummer : **85105449.4**

(22) Anmeldetag : **04.05.85**

(54) Vorrichtung zur transvenösen Einführung von Herzschrittmacher-Elektroden od. dgl.

(30) Priorität : **01.06.84 DE 3420455**

(43) Veröffentlichungstag der Anmeldung :
**04.12.85 Patentblatt 85/49**

(45) Bekanntmachung des Hinweises auf die Patenterteilung : **17.08.88 Patentblatt 88/33**

(84) Benannte Vertragsstaaten :
**AT CH FR GB IT LI NL**

(56) Entgegenhaltungen :
**GB-A- 704 488**
**US-A- 3 598 118**
**US-A- 3 677 244**
**US-A- 4 345 606**
**US-E- 31 855**

(73) Patentinhaber : **Osypka, Peter, Dr. Ing.**
**Basler Strasse 109**
**D-7889 Grenzach-Wyhlen (DE)**

(72) Erfinder : **Osypka, Peter, Dr. Ing.**
**Basler Strasse 109**
**D-7889 Grenzach-Wyhlen (DE)**

(74) Vertreter : **Patentanwälte Dipl.-Ing. Hans Schmitt**
**Dipl.-Ing. Wolfgang Maucher**
**Dreikönigstrasse 13**
**D-7800 Freiburg i.Br. (DE)**

**Beschreibung**

Die Erfindung betrifft eine Vorrichtung zur transvenösen Einführung von Herschrittmacher-Elektroden, Kathetern od. dgl. Sonden in das Herz oder das Kreislaufsystem, mit einer Metallkanüle zur Punktion insbesondere der Vena subclavia, mit einer durch die Metallkanüle einführbaren Metallspirale od. dgl. Führungssonde, mit einem Dilatator mit aufsitzender Hülse sus Kunststoff zum Einführen in die Vene über die Metallspirale hinweg, durch welche Hülse die Elektrode, der Katheter od. dgl. nach Zurückziehen der Metallspirale und des Dilatators in die Vene einführbar ist und die anschließend von der Elektrode mittels seitlicher Öffnung entfernbar ist.

Eine derartige Vorrichtung ist beispielsweise aus Herz/ Kreislauf 10, Nr. 10 (1978) 489 bis 494 « Permanente Herzschrittmacherversorgung mittels perkutaner Implantationstechnik » bekannt. Dabei wird so vorgegangen, daß die Punktion der Vena subclavia mit Hilfe der Metallkanüle erfolgt. Dadurch wird durch das Innenlumen dieser Metallkanüle eine bewegliche Metallspirale, die sogenannte Seldinger-Spirale, eingeführt. Die Metallkanüle kann hiernach herausgezogen werden, so daß zunächst nur die Metallspirale in der Vene verbleibt. Über diese Metallspirale wird der Dilatator mit aufsitzender KunststoffHülse — in der Regel unter leichten Drehbewegungen — in die Vene vorgeschoben. Unter Röntgen-Kontrolle werden Spirale und Dilatator entfernt und durch die verbleibende Hülse aus Kunststoff kann dann die Elektrode eingeführt und bis in das Herz vorgeschoben werden. Da am Ende der Herzschrittmacher-Elektrode ein Stecker zum Anschließen an den eigentlichen Herzschrittmacher vorgesehen ist, kann anschließend die Hülse nicht ohne weiteres von der Elektrode abgestreift werden. Gemäß der erwähnten Veröffentlichung muß entweder der Stecker abgeschnitten oder aber die Hülse mit der Schere in Längsrichtung gespalten werden, um durch die so entstehende seitliche Öffnung ein Abnehmen von der Elektrode zu erlauben.

Gemäß der US-PS 4 345 606 ist deshalb eine derartige Hülse bekannt, die an einer Seite vorgeschlitzt ist, um seitlich von der Elektrode abgenommen werden zu können. Dabei besteht jedoch der Nachteil, daß durch die anatomisch bedingte Krümmung die durch den Schlitz geschwächte Hülse leicht ein- oder abgeknickt werden kann. Wenn dies geschieht, ist die Hülse unbrauchbar geworden, so daß die Prozedur wiederholt werden muß. Ferner besteht beim seitlichen Abstreifen der Hülse die Gefahr, daß die bewußt weiche Isolation der Elektrode zwischen den Schlitzrändern der Hülse eingeklemmt und dadurch verletzt werden kann. Einerseits muß die Hülse so steif sein, daß sie trotz des sie schwächenden Längsschlitzes nicht abknickt, andererseits besteht bei zu steifer Hülse die Gefahr der Perforation oder bei Abstreifen der Hülse eine Verletzungsgefahr der Elektrode durch scharfe Schnittkanten.

Es ist auch schon eine Lösung gemäß US-PS 4 243 050 bekannt, bei welcher eine in Längsrichtung auseinanderziehbare Hülse mit jeweils zwei Längshälften vorgesehen ist, deren eine von der anderen teilweise umschlossen ist. Nach dem Auseinanderziehen dieser beiden Hülsenteile kann dann jeder Teil aufgrund seines genügend großen seitlichen Schlitzes von der Elektrode abgenommen werden. Der Nachteil dieser Vorrichtung besteht neben dem Herstellungsaufwand darin, daß während des Auseinanderziehens der Hülsenteile die Elektrode nicht sicher festgehalten werden kann, so daß zwischen Elektrode und Hülse unerwünscht Blut entweichen kann. Auch kommt es vor, daß die Hülsenteile nicht gleichmäßig in Längsrichtung auseinandergehen, so daß anschließend mit Hilfe einer Schere oder einem Skalpell die Reste vorsichtig abgeschnitten werden müssen. Dabei besteht wiederum die Gefahr von Verletzungen der Elektroden-Isolation.

Der Erfindung liegt die Aufgabe zugrunde, eine Vorrichtung der eingangs erwähnten Art zu schaffen, bei welcher die Vorteile der Subclavia-Punktion beibehalten werden können, die Hülse nicht von vorneherein mit einem seitlichen Schlitz versehen sein muß und dennoch ein leichtes Abnehmen der Hülse von der Elektrode ohne Abschneiden des Elektroden-Steckers möglich ist.

Zur Lösung dieser Aufgabe ist eine Vorrichtung bzw. ein Einführbesteck der eingangs erwähnten Art gekennzeichnet durch eine auf die Elektrode außerhalb der Hülse aufsetzbare Trenneinrichtung, die eine an der Elektrode angreifende Halterung und ein radial davon abstehendes Messer oder Skalpell aufweist, dessen Schneide gegen die Stirnseite der Hülse gerichtet ist.

Dadurch ist es möglich, beim Zurückziehen der Hülse für deren Entfernung aus der Vene nach dem Einsetzen einer Elektrode od. dgl. diese gleichzeitig durch diese Rückziehbewegung in Längsrichtung zu spalten. Ein Vorsichtiges Ansetzen einer Schere mit der entsprechenden Gefahr für die Elektroden-Isolierung oder eine aufwendige Ausbildung der Hülse aus zwei in Längsrichtung auseinanderziehbaren vorgespaltenen Teilen oder die Verwendung einer vorgespalteten Hülse mit der entsprechenden Schwächung werden auf wirkungsvolle und einfache Weise vermieden. Die Hülse kann hinsichtlich Biegbarkeit, Geschmeidigkeit, Abdichtung der punktierten Stelle und Führungsqualitäten für die Elektrode od. dgl. optimal ausgelegt sein, ohne daß Rücksicht auf die spätere Entfernbarkeit genommen werden muß. Dies geschieht dann durch die zusätzliche Trenneinrichtung, die auf der Elektrode aufsetzbar ist und mit der vorstehenden Schneide des Messers beim Zurückziehen die Hülse automatisch für ein seitliches Wegnehmen spaltet.

Besonders zweckmäßig ist es, wenn die Halterung für das Messer od. dgl. die Elektrode U-förmig oder C-förmig zumindest teilweise umschließt und in Längsrichtung der Elektrode Vorzugsweise eine größere Ausdehnung als das Messer hat. Die Halterung hat somit eine hülsenartige Form, kann aber von der Seite her auf die Elektrode aufgesetzt und auch gut an dieser festgehalten werden. Die größere Länge der Halterung erlaubt dabei eine gute Aufnahme der von dem Messer erzeugten Schnittkräfte.

Eine besonders zweckmäßige und Vorteilhafte Ausgestaltung der Erfindung kann darin bestehen, daß die Hülse an der dem Messer zugewandten Stirnseite wenigstens zwei etwa radial abstehende, vorzugsweise einstückig angebrachte Griffe od. dgl. Vorsprünge aufweist, die in Ausgangsposition einen Winkelabstand zueinander haben und für das Aufschlitzen der Hülse gegeneinander biegbar und zusammendrückbar sind. Durch das Zusammendrücken dieser Griffe am Ende der Kunststoff-Hülse wird somit eine Stelle markiert oder festgelegt, an der sich die Hülse mit Hilfe des Messers bei ihrem Zurückziehen am einfachsten aufschlitzen läßt. Somit haben diese Griffe eine Doppelfunktion, indem sie einerseits die Schneidstelle angeben und andererseits das Zurückziehen erleichtern.

Der Halter der Trenneinrichtung kann wenigstens an seinem der Hülse bzw. der Vene zugewandten Ende konisch sein bzw. eine kleinere Außenabmessung als die Innenhöhlung der Hülse haben und in diese abdichtend einsteckbar sein. Damit ergibt sich der zusätzliche Vorteil, daß der Halter auch während des Schlitzens, wenn die Hülse mit ihrer Innenhöhlung über diesen Halter gezogen wird, eine abdichtende Funktion ausübt und einen Blutaustritt zwischen Elektrode und Hülse so lange und so weit wie möglich unterbindet. Dabei kann unmittelbar an dem der Hülse zugewandten und in diese teilweise einführbaren Ende der Halterung der Trenneinrichtung die Messerschneide von der Halterung abgehen. Der Beginn des Trennvorganges fällt somit mit der Einführung der konischen Halterung in die Hülse zusammen.

Die radial an dem der Trenneinrichtung zugewandten Ende der Hülse vorgesehenen Handgriffe können flügelartig ausgebildet sein und auf den beim Zusammendrücken voneinander abgewandten Seiten Einbuchtungen haben. Dadurch finden die Finger des Benutzers eine gute Lagerung, so daß die Bedienung der Griffe vereinfacht ist, die einerseits zueinander bewegt und andererseits zusammen mit der Hülse in axialer Richtung der Elektrode zurückgezogen werden müssen.

Zur Schonung der Elektrode kann die Halterung der Trenneinrichtung aus federndem Werkstoff bestehen und an die Elektrode andrückbar sein. Dadurch verteilen sich die Haltekräfte gut über die Länge der Halterung, so daß keine Gefahr für die Elektroden-Isolierung besteht. Dennoch kann der Benutzer auf einfache Weise die Halterung festhalten, indem er sie leicht zusammendrückt.

Vor allem bei Kombination einzelner oder aller der vorbeschriebenen Merkmale und Maßnahmen ergibt sich eine Vorrichtung zum transvenösen Einführen von Herzschrittmacher-Elektroden od.

dgl. bzw. ein Einführbesteck, bei welchem das Ein- oder Abknicken der zugehörigen Hülse durch Vermeidung eines schwächenden vorgefertigten Schlitzes verhindert werden kann. Vielmehr kann die Konstruktion der Hülse unabhängig davon, wie sie später entfernt wird, ausschließlich nach den anatomischen Erfordernissen ausgelegt werden. Dennoch kann sie anschließend durch die erfindungsgemäße Trenneinrichtung exakt, sicher und zwangsläufig aufgeschlitzt und somit leicht abgestreift werden. In vorteilhafter Weise wird dabei ein Blutverlust auch durch diese Trenneinrichtung minimalisiert, weil diese an der der Vene abgewandten Mündung der Hülse abdichtend angesetzt werden kann. Ein aufwendiges und schwieriges Aufschlitzen der Hülse mit einer Schere oder das Abschneiden des Steckers werden vermieden.

Nachstehend ist die Erfindung mit ihren ihr als wesentlich zugehörenden Einzelheiten anhand der Zeichnung noch näher beschrieben. Es zeigt in zum Teil schematisierter Darstellung :

Fig. 1 die Punktion der Vene mit Hilfe einer Metallkanüle, in vergrößertem Maßstab

Fig. 2 einen Längsschnitt durch die Vene mit eingesteckter Punktionskanüle,

Fig. 3 das Einführen einer beweglichen Metallspirale durch die Punktionskanüle hindurch in das Innere der Vene,

Fig. 4 das Entfernen der Metallkanüle durch Zurückziehen aus der Vene, wobei die Metallspirale in der Vene verbleibt,

Fig. 5 das Einbringen eines Dilatators mit aufsitzender Hülse aus Kunststoff über die Metallspirale hinweg in die Vene, wobei der Dilatator die Hülse von innen abstützt,

Fig. 6 das Zurückziehen des Dilatators und der Metallspirale bzw. des Führungsdrahtes aus der Hülse heraus, die alleine in der Vene verbleibt,

Fig. 7 den Beginn der Einführung der eigentlichen Elektrode durch die Hülse in die Vene und bis in das Herz,

Fig. 8 die Elektrode im Bereich der Führungshülse nach dem Einführen in die Vene,

Fig. 9 die Vorbereitung der Zurückziehung der Hülse durch Zusammendrücken zweier an ihrem äußeren Ende befindlicher Handgriffe,

Fig. 10 das Aufsetzen einer Trennvorrichtung gemäß Fig. 11 auf die verbleibende Elektrode, gegen welche die Hülse zum Aufschlitzen zurückziehbar ist,

Fig. 11 in schaubildlicher und etwas vergrößerter Darstellung die Trennvorrichtung mit einem abstehenden Messer, dessen Schneide gegen die zurückzuziehende Hülse gerichtet ist,

Fig. 12 in schaubildlicher Darstellung die vor Gebrauch ungeschlitzte durchgehende Hülse und

Fig. 13 den Vorgang des Entfernens der Hülse, die an ihren Griffen erfaßt und gegen die mit der anderen Hand fixierte Trenneinrichtung gezogen wird, wodurch sie aus der Vene entfernt und dabei gleichzeitig in Längsrichtung geschlitzt wird, so daß sie seitlich von der Elektrode abgenommen werden kann.

Eine Vorrichtung zum transvenösen Einführen beispielsweise einer in den Figuren 7 bis 10 teilweise erkennbaren Herzschrittmacher-Elektrode 1 in eine Vene 2, durch welche sie dann in das Herz oder Kreislaufsystem eingeschoben werden kann, weist mehrere teils zusammen teils nacheinander einzusetzende, im folgenden näher zu beschreibende Teile auf, die zusammen ein sogenanntes Einführungsbesteck bilden.

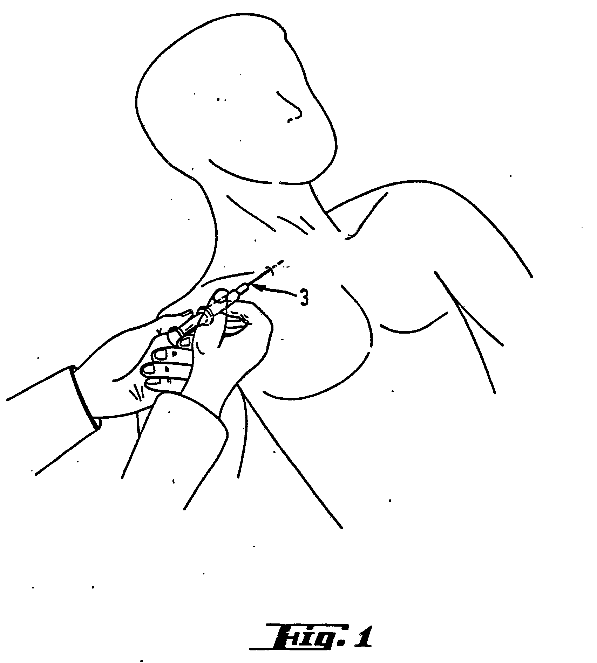

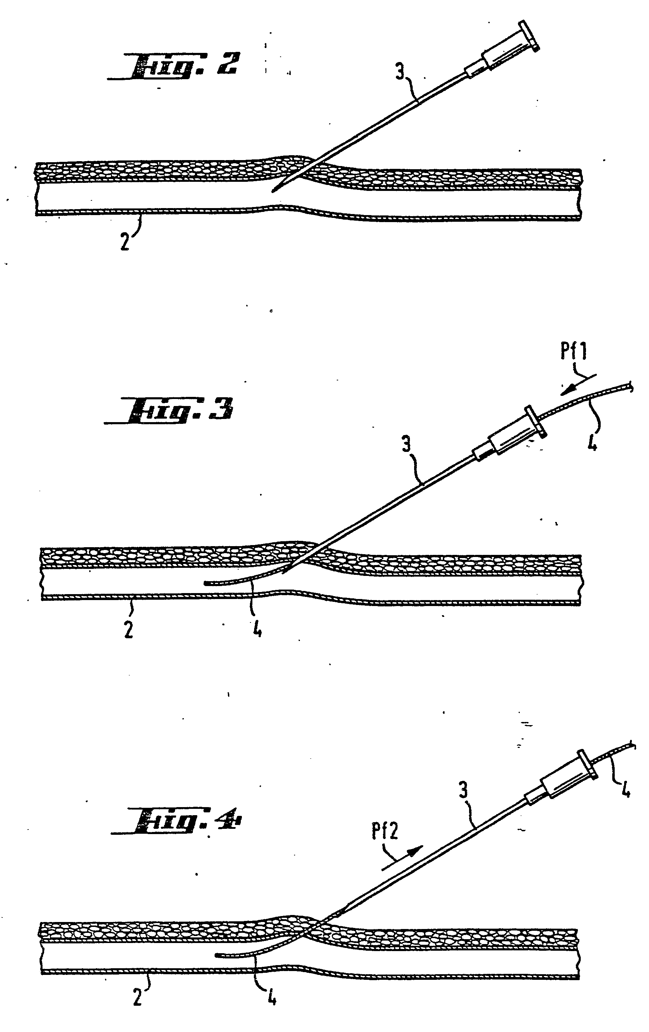

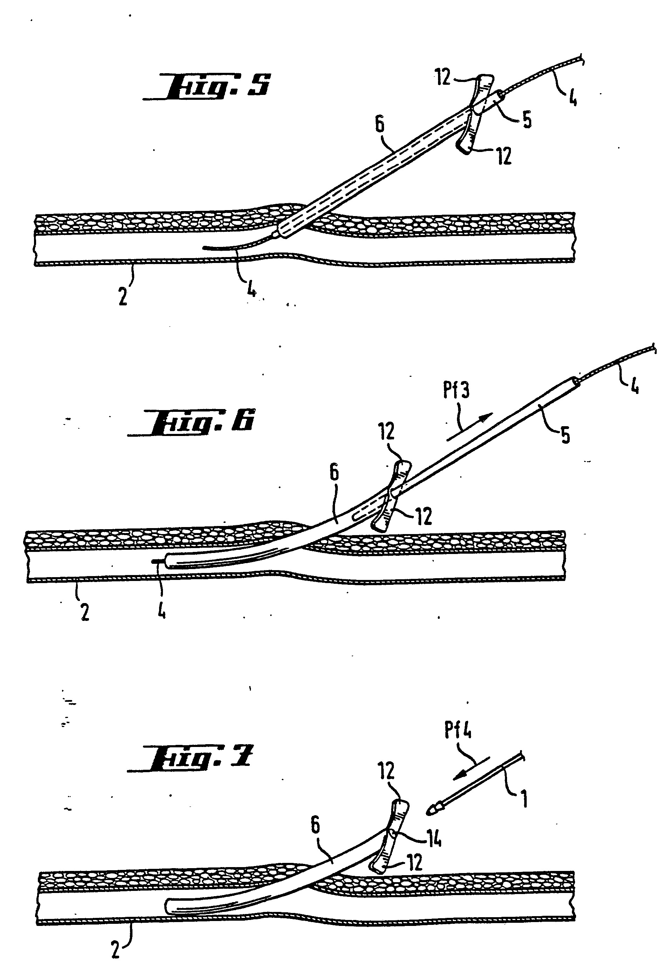

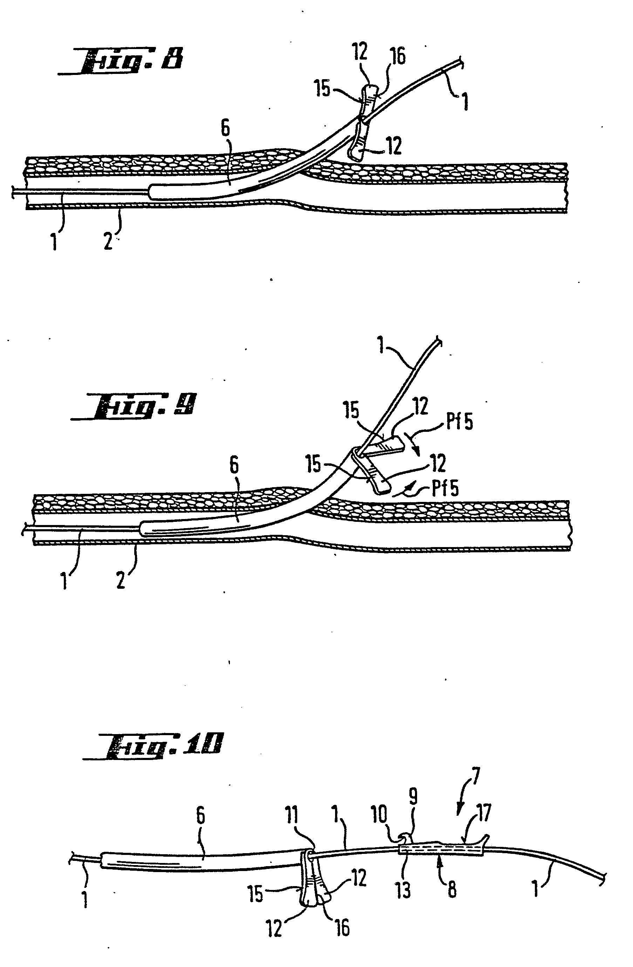

In den Figuren 1 und 2 erkennt man eine Kanüle 3, mit der die Vene 2 punktiert wird. In Fig. 1 wird die Kanüle 3 gerade angesetzt, während sie in Fig. 2 die Punktion durchgeführt hat. Anschließend kann gemäß Fig. 3 durch diese Kanüle 3 eine Führungssonde, beispielsweise eine Metallspirale 4 in Richtung des Pfeiles Pf 1 in die Vene 2 eingeführt werden. Danach wird gemäß Fig. 4 die Kanüle 3 entsprechend dem Pfeil Pf 2 aus der Vene 2 zurückgezogen, während die Metallspirale 4 als Führungssonde in der Vene 2 verbleibt.

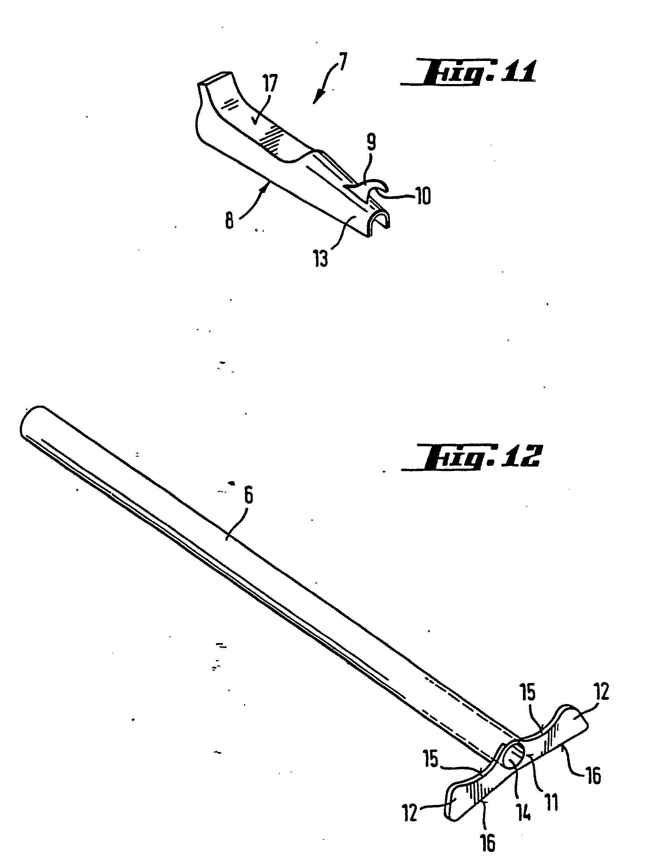

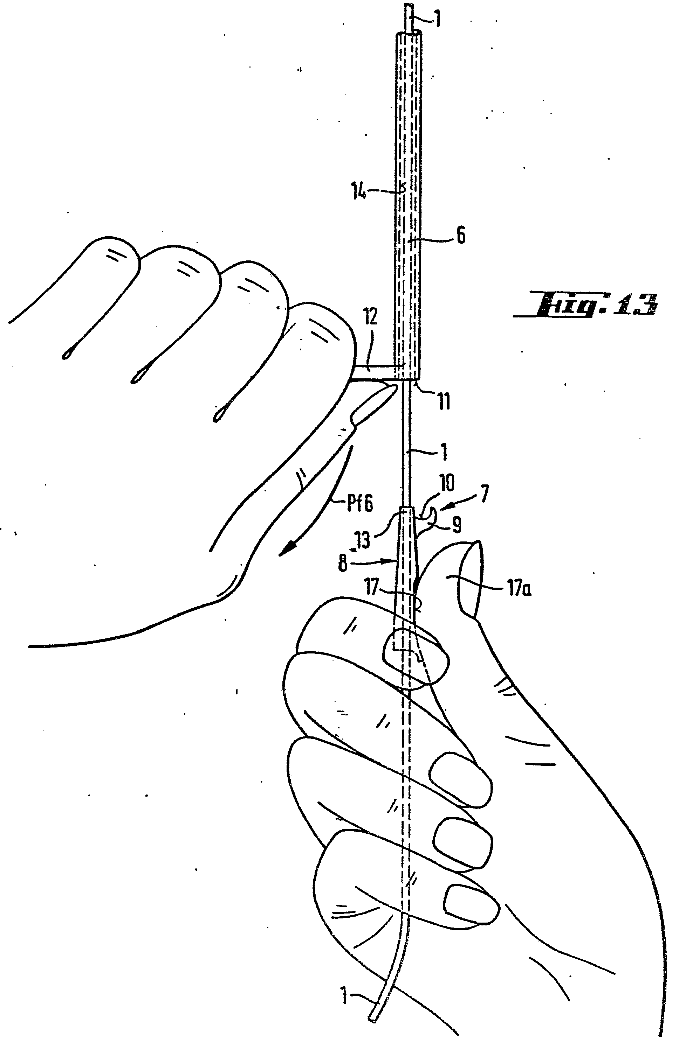

Ferner gehört zu der Vorrichtung ein Dilatator 5, auf welchem zunächst eine Hülse 6 aus Kunststoff sitzt. Der Dilatator 5 wird zusammen mit dieser Hülse 6 gemäß Fig. 5 über die Metallspirale 4 teilweise in die Vene 2 eingeführt. Anschließend kann gemäß Fig. 6 der die Hülse 6 beim Einführen in die Vene von innen stützende Dilatator gemäß 5 dem Pfeil Pf 3 zusammen mit dem Führungsdraht bzw. der Metallspirale 4 aus der Verbleibenden Hülse 6 zurückgezogen werden. Die bis dahin von dem Dilatator 5 gestützte und entsprechend ausgesteifte Hülse 6 kann nun, was in Fig. 6 und 7 angedeutet ist, gut etwas gekrümmt werden, um eine sichere Führungsbahn für die durch die Hülse 6 einzuführende Elektrode 1 zu bilden. In Fig. 7 ist angedeutet, wie das Vordere Ende der Elektrode 1 entsprechend dem Pfeil Pf 4 von außen her in die Hülse 6 eingesteckt wird. In Fig. 8 und 9 erkennt man, wie die Elektrode 1 dann durch die Hülse 6 hindurch in die Vene 2 eingeführt ist, durch die sie mit ihrem vorderen Ende beispielsweise in das Herz geschoben werden kann.

Wenn die Elektrode 1 bestimmungsmäßig verankert ist, muß anschließend noch die Hülse 6 entfernt werden. Ein einfaches Zurückziehen über die Elektrode 1 hinweg nach hinten wird jedoch durch den dort befindlichen, in der Zeichnung nicht dargestellten Elektroden-Stecker verhindert, dessen Außenabmessungen die der Hülse 6 übertreffen und der zum Verbinden mit dem eigentlichen Herzschrittmacher dient. Bei der Vorrichtung bzw. dem Einführbesteck gemäß der Erfindung ist deshalb eine in Fig. 11 deutlicher dargestellte Trenneinrichtung 7 Vorgesehen, mit deren Hilfe die Hülse 6, die in Fig. 12 noch verdeutlicht dargestellt ist, bei ihrem Zurückziehen gemäß Fig. 13 gleichzeitig in Längsrichtung aufgeschlitzt werden kann. Die Trenneinrichtung 7 kann dabei gemäß Fig. 10 und 13 auf die Elektrode 1 außerhalb der Hülse 6 aufgesetzt werden, wofür sie eine an der Elektrode 1 angreifende Halterung 8 und ein radial davon abstehendes Messer oder Skalpell 9 aufweist, dessen Schneide 10 in Gebrauchsstellung gegen die Stirnseite 11 der Hülse 6 gerichtet ist. Dabei ist die Halterung 8 für das Messer 9 U-förmig oder C-förmig ausgebildet, so daß sie die Elektrode 1 teilweise umschließen kann. In Längsrichtung hat dabei diese Halterung 8 eine relativ große Ausdehnung, die dieses Messer 9 erheblich übertrifft, so daß die Halterung 8 in ihrer Gebrauchsstellung sicher mit einer Hand festgelegt werden kann, wie Fig. 13 es zeigt.

Die Hülse 6 hat an der dem Messer 9 zugewandten Stirnseite 11 wenigstens zwei etwa radial abstehende, im Ausführungsbeispiel einstückig angebrachte Griffe 12, die in Ausgangsposition einen relativ großen Winkelabstand zueinander haben und für das Aufschlitzen der Hülse 6 gemäß den beiden zueinander gerichteten bogenförmigen Pfeilen Pf 5 in Fig. 9 gegeneinander biegbar und gemäß Fig. 10 und 13 zusammendrückbar sind. In dieser in Fig. 10 und 13 dargestellten Position kann nun mit der anderen Hand die Hülse 6 gemäß dem Pfeil Pf 6 einerseits zurückgezogen und andererseits auch seitlich von der Elektrode 1 weggezogen werden, weil die Hülse 6 beim Zurückziehen von dem Messer 9 und dessen Schneide 10 gegenüber den beiden Griffen 12 an einer durch das Zusammenbiegen der Griffe unter Zugspannung stehenden Stelle aufgeschlitzt wird, wobei gerade die Zugkraft am Anfang der Stirnseite der Hülse 6 das Einschneiden und Aufspalten der Hülse erleichtert.

Man erkennt vor allem in den Figuren 11 und 13, daß der Halter 8 der Trenneinrichtung 7 wenigstens an seinem der Hülse 6 bzw. der Vene 2 zugewandten Ende 13 konisch ist bzw. eine kleinere Außenabmessung als die Innenhöhlung 14 der Hülse 6 hat und in diese also abdichtend einsteckbar ist. Dadurch wird beim Entnehmen der Hülse 6 ein zu großer Austritt von Blut verhindert, weil die Halterung 8 und ihr konisches Ende 13 zusätzlich eine Abdichtung bewirkt.

Da praktisch unmittelbar an dem der Hülse 6 zugewandten und in diese teilweise einführbaren Ende 13 der Halterung 8 der Trenneinrichtung 7 oder mit geringem Abstand dazu die Messerschneide 10 von der Halterung 8 abgeht, fällt der Eintritt des konischen Endes 13 in die Innenhöhlung 14 der Hülse 8 praktisch mit dem Beginn des Trennvorganges zusammen. Es wird somit entsprechend wenig Zeit verloren. Gleichzeitig bewirkt dabei das konische Ende 13 noch eine gewisse Führung und Stützung der Hülse 6 im Bereich des durchzuführenden Trennschnittes, so daß das Messer 9 sicher angesetzt werden kann und gut in die Hülse einschneiden kann.

Vor allem in Fig. 12 erkennt man deutlich, daß die radial an dem der Trenneinrichtung 7 zugewandten Ende der Hülse 6 vorgesehenen Handgriffe 12 flügelartig ausgebildet sind und auf den beim Zusammendrücken voneinander abgewandten Seiten Einbuchtungen 15 für die Finger des Benutzers haben. Die zusammendrückbaren Seiten 16 sind hingegen praktisch geradlinig, um sich gemäß Fig. 10 gut aneinanderlegen und zusammendrücken zu lassen.

Die Halterung 8 der Trenneinrichtung 7 besteht zweckmäßigerweise aus federndem Werkstoff, so daß sie an der Elektrode 1 von der Hand des Benutzers andrückbar ist und so die auftretenden Schneidkräfte ohne ungewollte Verschiebung aufnehmen kann. Dabei erkennt man vor allem in den Figuren 10 und 11, daß die Halterung 8 hinter dem Messer 9 eine Gruffmulde 17 od. dgl. Verformung aufweist, in die zur Aufnahme einer entsprechenden Gegenkraft der Daumen 17 a des Benutzers paßt und eingelegt werden kann. Gegebenenfalls kann dadurch sogar erforderlichenfalls die Trenneinrichtung 7 auch noch etwas in Richtung auf die Hülse 6 hin verschoben werden.

Wird also die Hülse 6 aus ihrer in den Figuren 8 und 9 dargestellten Position nach dem Zusammendrücken der Griffe 12 gemäß Fig. 10 entsprechend dem Pfeil Pf 6 in Fig. 13 zurückgezogen, kann sie gegen die Trenneinrichtung 7 gezogen werden, wodurch sie gleichzeitig an der Seite aufgeschlitzt wird. Mit dem Fortschritt dieses Schlitzes beim Zurückziehen der Hülse kann sie gleichzeitig auch seitlich weggezogen werden, so daß auf relativ engem Raum nicht nur das Zurückziehen der Hülse 6, sondern auch ihr Entfernen von der Elektrode 1 erfolgen kann. Dadurch ist es möglich, daß diese Hülse 6 zunächst bei ihrer Führungsfunktion gemäß Fig. 7 eine genügend steife und stabile Ausbildung hat, die ein das Einführen der Elektrode 1 verhinderndes Abknicken vermeidet, dennoch aber später leicht ohne Behinderung durch den Elektroden-Stecker entfernt werden kann.

Alle in der Beschreibung, den Ansprüchen, der Zusammenfassung und der Zeichnung dargestellten Merkmale und Konstruktionsdetails können sowohl einzeln als auch in beliebiger Kombination miteinander wesentliche Bedeutung haben.

**Patentansprüche**

1. Vorrichtung zur transvenösen Einführung von Herzschrittmacher-Elektroden (1), Kathetern od. dgl. Sonden in das Herz oder das Kreislaufsystem, mit einer Metallkanüle (3) zur Punktion insbesondere der Vena subclavia, mit einer durch die Metallkanüle (3) einführbaren Metallspirale (4) od. dgl. Führungssonde, mit einem Dilatator (5) mit aufsitzender Hülse (6) aus Kunststoff zum Einführen in die Vene über die Metallspirale (4) hinweg, durch welche Hülse (6) die Elektrode (1), der Katheter od. dgl. nach Zurückziehen der Metallspirale (4) und des Dilatators (5) in die Vene einführbar ist und die anschließend von der Elektrode (1) mittels seitlicher Öffnung entfernbar ist, gekennzeichnet durch eine auf die Elektrode (1) außerhalb der Hülse (6) aufsetzbare Trenneinrichtung (7), die eine an der Elektrode (1) angreifende Halterung (8) und ein radial davon abstehendes Messer oder Skalpell (9) aufweist, dessen Schneide (10) gegen die Stirnseite (11) der Hülse (6) gerichtet ist.

2. Vorrichtung nach Anspruch 1, dadurch gekennzeichnet, daß die Halterung (8) für das Messer (9) od. dgl. die Elektrode (1) U-förmig oder C-förmig zumindest teilweise umschließt und in Längsrichtung der Elektrode vorzugsweise eine größere Ausdehnung als das Messer (9) hat.

3. Vorrichtung nach Anspruch 1 oder 2, dadurch gekennzeichnet, daß die Hülse (6) an der dem Messer (9) zugewandten Stirnseite (11) wenigstens zwei etwa radial abstehende, vorzugsweise einstückig angebrachte Griffe (12) od. dgl. Vorsprünge aufweist, die in Ausgangsposition einen Winkelabstand zueinander haben und für das Aufschlitzen der Hülse (6) gegeneinander biegbar und zusammendrückbar sind.

4. Vorrichtung nach einem der Ansprüche 1 bis 3, dadurch gekennzeichnet, daß der Halter (8) der Trenneinrichtung (7) wenigstens an seinem der Hülse (6) bzw. der Vene (2) zugewandten Ende (13) konisch ist bzw. eine kleinere Außenabmessung als die Innenhöhlung (14) der Hülse (6) hat und in diese abdichtend einsteckbar ist.

5. Vorrichtung nach einem der Ansprüche 1 bis 4, dadurch gekennzeichnet, daß an dem der Hülse (6) zugewandten und in diese teilweise einführbaren Ende (13) der Halterung (8) der Trenneinrichtung (7) die Messerschneide (10) von der Halterung (8) abgeht.

6. Vorrichtung nach einem der Ansprüche 1 bis 5, dadurch gekennzeichnet, daß die radial an dem der Trenneinrichtung (7) zugewandten Ende der Hülse (6) vorgesehenen Handgriffe (12) flügelartig ausgebildet sind und auf den beim Zusammendrücken voneinander abgewandten Seiten Einbuchtungen (15) haben.

7. Vorrichtung nach einem der Ansprüche 1 bis 6, dadurch gekennzeichnet, daß die Halterung (8) der Trenneinrichtung (7) aus federndem Werkstoff besteht und an der Elektrode (1) andrückbar ist.

8. Vorrichtung nach einem der Ansprüche 1 bis 7, dadurch gekennzeichnet, daß die Halterung (8) hinter dem Messer (9) eine Griffmulde (17) od. dgl. Verformung aufweist.

9. Vorrichtung nach einem der Ansprüche 1 bis 8, dadurch gekennzeichnet, daß die beim Zusammendrücken gegeneinanderliegenden Seiten (16) der Griffe (12) geradlinig sind.

**Claims**

1. An appliance for the transveinous introduction of pacemaker electrodes (1), catheters or similar probes into the heart or the circulatory system, including a metal cannula (3) for puncture, in particular of the vena subclavia, including a metal coil (4) or similar guide probe adapted to be introduced through the metal cannula (3), including a dilator (5), upon which a plastic sleeve (6) is seated, for introduction into the vein by way of the metal coil (4), the electrode (1), the catheter or the like being adapted to be introduced into the vein through said sleeve (6) after the metal coil (4) and the dilator (5) have been withdrawn and said sleeve subsequently being removable from the electrode (1) by means of a lateral opening, characterized by a parting means (7) which is adapted to be mounted upon the electrode (1) outside the sleeve (6) and has a holder (8) engaging the electrode (1) and a knife or scalpel (9) projecting radially therefrom, the cutting edge (10) of the latter being directed towards the end face (11) of the sleeve (6).

2. The appliance as claimed in claim 1, characterized in that the holder (8) for the knife (9) or the like at least partly embraces the electrode (1) in a U or C shape and in the longitudinal direction of the electrode preferably has a larger expanse than the knife (9).

3. The appliance as claimed in claim 1 or claim 2, characterized in that the sleeve (6) has on the end face (11) facing the knife (9) at least two handles (12) or similar projections which protrude approximately radially and are preferably attached in one piece, in the starting position said handles being at an angular distance from each other and being adapted to be bent towards each other and urged together for slitting open the sleeve (6).

4. The appliance as claimed in any one of claims 1 to 3, characterized in that the holder (8) of the parting means (7) is conical at least at its end (13) facing the sleeve (6) and the vein (2), i. e. has a smaller external dimension than the inside cavity (14) of the sleeve (6), and is adapted to be sealingly inserted into the latter.

5. The appliance as claimed in any one of claims 1 to 4, characterized in that the knife-edge (10) departs from the holder (8) at that end (13) of the holder (8) of the parting means (7) which faces the sleeve (6) and is adapted to be partly introduced into the latter.

6. The appliance as claimed in any one of claims 1 to 5, characterized in that the handles (12) provided radially at the sleeve (6) end facing the parting means (7) are devised to be winglike and have indentations (15) on the sides which are averted from each other when the handles are urged together.

7. The appliance as claimed in any one of claims 1 to 6, characterized in that the holder (8) of the parting means (7) is made of springy material and is adapted to be pressed against the electrode (1).

8. The appliance as claimed in any one of claims 1 to 7, characterized in that the holder (8) has a grip-type depression (17) or similar deformation behind the knife (9).

9. The appliance as claimed in any one of claims 1 to 8, characterized in that the sides (16) lying against each other when the handles (12) are urged together run in a straight line.

### Revendications

1. Dispositif destiné à l'introduction intraveineuse, dans le cœur ou le système circulatoire, d'électrodes (1) de stimulateurs cardiaques, de cathéters ou sondes similaires, comprenant une canule métallique (3) destinée notamment à ponctionner la veine subclaviculaire, une spirale métallique (4) ou sonde de guidage similaire pouvant être introduite par l'intermédiaire de la canule métallique (3), un dilatateur (5) sur lequel est enfilée une gaine (6) en matière plastique, en vue de l'insertion dans la veine à travers la spirale métallique (4), gaine (6) par l'intermédiaire de laquelle l'électrode (1), le cathéter ou instrument similaire peut être introduit dans la veine après le retrait de la spirale métallique (4) et du dilatateur (5), ladite gaine pouvant ensuite être enlevée de l'électrode (1) au moyen d'une ouverture latérale, caractérisé par un dispositif séparateur (7) qui peut être implanté sur l'électrode (1) à l'extérieur de la gaine (6), et présente une pièce de retenue (8) venant en prise avec l'électrode (1), ainsi qu'une lame ou scalpel (9) qui en fait saillie radialement et dont le tranchant (10) est dirigé vers la face frontale (11) de la gaine (6).

2. Dispositif selon la revendication 1, caractérisé par le fait que la pièce de retenue (8) associée à la lame (9) ou organe analogue entoure au moins en partie l'électrode (1), en forme de U ou en forme de C, et présente de préférence, dans le sens longitudinal de l'électrode, une plus grande étendue que la lame (9).

3. Dispositif selon la revendication 1 ou 2, caractérisé par le fait que la gaine (6) comporte, à la face frontale (11) tournée vers la lame (9), au moins deux plaquettes de préhension (12) ou saillies similaires qui débordent sensiblement dans le sens radial, sont ménagées de préférence d'un seul bloc, sont espacées angulairement l'une de l'autre dans la position initiale, et peuvent être ployées et comprimées l'une contre l'autre pour ouvrir la gaine (6) par incision.

4. Dispositif selon l'une des revendications 1 à 3, caractérisé par le fait que la pièce de retenue (8) du dispositif séparateur (7) est conique, au moins à son extrémité (13) respectivement tournée vers la gaine (6) ou la veine (2), ou possède respectivement une dimension externe plus petite que la cavité interne (14) de la gaine (6), et peut être emboîtée dans cette dernière avec effet d'étanchéité.

5. Dispositif selon l'une des revendications 1 à 4, caractérisé par le fait que le tranchant (10) de la lame fait saillie de la pièce de retenue (8) à l'extrémité (13) de cette pièce de retenue (8) du dispositif séparateur (7) qui est tournée vers la gaine (6) et peut être partiellement introduite dans celle-ci.

6. Dispositif selon l'une des revendications 1 à 5, caractérisé par le fait que les plaquettes de préhension (12), prévues radialement sur l'extrémité de la gaine (6) tournée vers le dispositif séparateur (7), sont réalisées en forme d'ailes et présentent des échancrures (15) vers l'intérieur, sur les côtés tournés à l'opposé l'un de l'autre lors de la compression.

7. Dispositif selon l'une des revendications 1 à 6, caractérisé par le fait que la pièce de retenue (8) du dispositif séparateur (7) consiste en un matériau élastique et peut être pressée contre l'électrode (1).

8. Dispositif selon l'une des revendications 1 à 7, caractérisé par le fait que la pièce de retenue (8) présente, derrière la lame (9), une zone de préhension encaissée (17) ou une déformation similaire.

9. Dispositif selon l'une des revendications 1 à 8, caractérisé par le fait que les côtés (16) des plaquettes de préhension (12), qui sont appliqués l'un contre l'autre lors de la compression, sont rectilignes.

**Fig. 1**

**Fig. 2**

3

2

**Fig. 3**

Pf1

3

4

2

4

**Fig. 4**

3

4

Pf2

2

4

**Fig. 5**

12

6

5

4

12

2

4

**Fig. 6**

Pf 3

12

6

5

4

12

2

4

**Fig. 7**

Pf 4

12

6

1

14

12

2

**Fig. 8**

**Fig. 9**

**Fig. 10**

**Fig. 11**

**Fig. 12**

Fig. 13